# EUROPÄISCHE PATENTSCHRIFT

(11) **EP 0 158 927 B1**
(45) Veröffentlichungstag und Bekanntmachung des Hinweises auf die Patenterteilung: **08.12.1993**
(21) Anmeldenummer: 85104028.7
(22) Anmeldetag: 03.04.1985
(51) Int. Cl.: A61K 37/02, A61K 37/64

(54) **Verfahren zur Behandlung der Herzinsuffizienz**
Process for the treatment of heart failure
Procédé pour traitement d'insuffisance cardiaque

(30) Priorität: 12.04.1984 DE 3413710
(43) Veröffentlichungstag der Anmeldung: 23.10.1985
(60) Teilanmeldung: 93102949.0
(73) Patentinhaber: HOECHST AKTIENGESELLSCHAFT, 65926 Frankfurt am Main (DE)
(72) Erfinder: Henning, Rainer, Dr., D-6234 Hattersheim am Main (DE); Urbach, Hansjörg, Dr., D-6242 Kronberg/Taunus (DE); Teetz, Volker, Dr., D-6238 Hofheim am Taunus (DE); Geiger, Rolf, Prof. Dr., D-6000 Frankfurt am Main 50 (DE); Schölkens, Bernward, Dr., D-6233 Kelkheim (Taunus) (DE)

(56) Entgegenhaltungen:
- EP-A- 0 049 658
- EP-A- 0 051 301
- EP-A- 0 065 301
- EP-A- 0 079 022
- EP-A- 0 084 164
- US-A- 4 374 829
- Chemical Abstracts, Band 100, Nr19, 7. Mai 1984, Seite 34, Ref. Nr. 150853c, Columbus, Ohio, US. J Xiang etal.: "Influence of the novel non-sulfhydrl converting enzyme-inhibitor Hoe-489 on isolated rat hearts"
- Chemical Abstracts, Band 96, Nr. 17, 26. April 1982, Seite 58, Ref Nr. 135574t, Columbus, Ohio, US. B H Clappison et al.: "Role of the kallikrein-kimin system in the renal effects og angiotensin-converting enzyme inhibition in anesthetized dogs"
- Chemical Abstracts, Band 96, Nr. 11, 15 März 1982, Seite 129, Ref. Nr.80467c, Columbus, Ohio, US. T Saldeen et al.: "A peptide derived from fibrin(ogen) inhibits angiotensin converting enzyme and potentiates the effects of bradykinin"
- Chemical Abstracts, Band 92, Nr.19, 12. Mai 1980, Seite 388, Ref. Nr. 161393v, Columbus, Ohio, US. S Adigun et al.: "Effects of angiotensin converting enzyme inhibitor on cardiovascular response to lower body negative pressure"
- Biological Abstracts/RRM,Type 19/4/27, Ref. Nr. 71033136, Philadelphia, PA, US. K M Mullane et al.: "Prostacyclin mediates the potentiated hypotensive effect of bradykinin following captopril treatment"
- Biological Abstracts/RRM, Type 19/4/7, Ref. Nr. 77061589, Philadelphia, PA,US. W B Dunknan et al.: "Enalapril MK-421 a new angiotensin converting enzyme inhibitor acute and chronic effects in heart failure"
- Biological Abstracts/RRM, Type 19/4/6, Ref. Nr. 77077749, Philadelphia, PA, US. S Morioka et al.: "Cardiac and hormonal effects of enalapril in hypertension"
- Chemical Abstracts, Band 101, Nr. 5, 30. Juli 1984, Seite 33, Ref. Nr. 32993w, Columbus, Ohio, US. J Xiang et al.: "Effect of angiotensin-converting enzyme inhibitor Hoe-498 on noradrenaline-induced vasoconstriction in isolated perfused mesentery of rats"

## Beschreibung

Die Erfindung betrifft ein Verfahren zur Behandlung der Herzinsuffizienz durch perorale oder parenterale Anwendung von Angiotensin-Converting-Enzym-Inhibitoren der Formel I 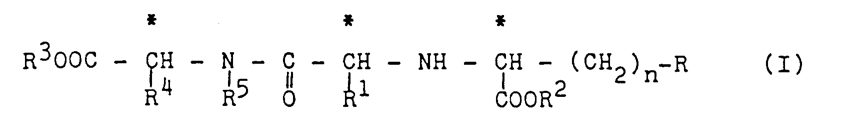
in welcher
- n=: 1 oder 2 ist,
- R=: Phenyl
- R¹: Wasserstoff oder (C₁-C₆)-Alkyl, das gegebenenfalls durch Amino substituiert sein kann,
- R² und R³: gleich oder verschieden sind und Wasserstoff oder Ethyl bedeuten und
- R⁴ und R⁵: zusammen mit den sie tragenden Atomen ein Octahydrocyclopenta[b]pyrrol System mit 5 bis 15 C-Atomen bilden.

Bei den Verbindungen, die mehrere chirale Atome besitzen, kommen alle möglichen Diastereomere als Racemate oder Enantiomere, oder Gemische verschiedener Diastereomere in Betracht.

Die in Betracht kommenden cyclischen Aminosäureester weisen die folgenden Strukturformeln auf. 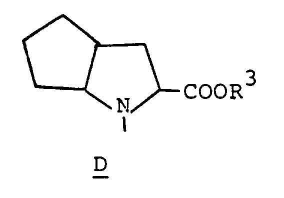
Insbesondere bevorzugt ist die Anwendung von Verbindungen der Formel I, in welcher n= 2 ist, R= Phenyl, R¹= Methyl.

Besonders vorteilhaft können die folgenden Verbindungen nach der erfindungsgemäßen Methode angewendet werden:
N-(1-S-Carbethoxy-3-phenyl-propyl)-S-alanyl-cis-endo-2-azabicyclo[3.3.0]octan-3-S-carbonsäure
N-(1-S-Carbethoxy-3-phenyl-propyl)-S-lysyl-cis-endo-2-azabicyclo[3.3.0]octan-3-S-carbonsäure
N-(1-S-Carbethoxy-butyl)-S-alanyl-cis-endo-2-azabicyclo[3.3.0]octan-3-S-carbonsäure
N-(1-S-Carbethoxy-3-phenyl-propyl)-S-lysyl-(2S,3aR,6aS)-octahydrocyclopenta[b]pyrrol-2-carbonsäure
N-(1-S-Carbethoxy-3-phenyl-propyl)-S-alanyl-2-(2S,3aR,6aS)-octahydrocyclopenta[b]pyrrol-2-carbonsäure
Diese Verbindungen lassen sich z.B. nach dem in der deutschen Patentanmeldung P 33 33 455.2 beschriebenen Verfahren herstellen, in dem die in der Anmeldung beschriebenen tert. Butyl- oder Benzylreste in bekannter Weise durch saure oder alkalische Hydrolyse oder durch Edelmetall-katalysierte Hydrogenolyse in die Monocarbonsäurederivate überführt werden. Die N^{ε}-Benzyloxycarbonylschutzgruppe der Lysinderivate wird durch Edelmetallkatalysierte Hydrogenolyse entfernt. Die oben aufgeführten Verbindungen lassen sich leicht mit physiologisch verträglichen Säuren oder Basen (im Falle von Mono- oder Dicarbonsäuren) in die entsprechenden Salze (z.B. Hydrochloride, Maleinate, Fumarate, etc.) überführen und als Salze die erfindungsgemäße Verwendung finden.

Die Verbindungen der Formel I sind Hemmstoffe des Angiotensin Converting Enzymes (ACE) beziehungsweise Zwischenprodukte bei der Herstellung von solchen Hemmstoffen und können auch zur Bekämpfung des Bluthochdrucks verschiedener Genese eingesetzt werden. Die Verbindungen der Formel I sind bekannt aus DE-OS 3211397, DE-OS 3227055, E-PA-46953, E-PA-79022, E-PA-84164, E-PA89637, E-PA-90362. Sie sind weiterhin Gegenstand der deutschen Patentanmeldungen P 32 42 151.6, P 32 46 503.3, P 32 46 757.5, P 33 00 774.8 und P 33 24 263.1.

In Ausübung der erfindungsgemäßen Methode können die oben beschriebenen Angiotensin Converting Enzyme-Inhibitoren an Säugern wie Affen, Hunden, Katzen, Ratten, Menschen etc. angewendet werden. Die für die erfindungsgemäße Verwendung geeigneten Verbindungen werden zweckmäßig in üblicher Weise in pharmazeutische Präparate eingearbeitet. Sie können in die üblichen Verabreichungsformen, wie Kapseln, Tabletten, Dragees, Lösungen, Salben, Emulsionen und auch in Depot-Form gebracht werden. Der Wirkstoff kann gegebenenfalls auch in mikroverkapselter Form vorliegen. Die Präparate können verträgliche, organische bzw. anorganische Begleitstoffe, beispielsweise Granulierstoffe, Klebe- und Bindemittel, Gleitmittel, Suspendiermittel, Lösungsmittel, antibakterielle Mittel, Netzmittel und Konservierungsmittel enthalten. Orale und parenterale Anwendungsformen werden bevorzugt. Die Verbindungen der Formel I können in Dosierungen von 0,1 - 50 mg pro Dosis ein- bis dreimal täglich verabreicht werden.

Die Wirksamkeit der Verbindungen der Formel I bei verschiedenen Formen der Herzinsuffizienz kann aus ihrer Wirkung in verschiedenen Testmodellen abgeleitet werden. Als Beispiele sollen im folgenden jeweils die Ergebnisse mit N-(1-S-Carbethoxy-3-phenyl-propyl)-S-alanyl-cis-endo-2-azabicyclo[3,3,0]octan-3-S-carbonsäure (Formel II) dienen. 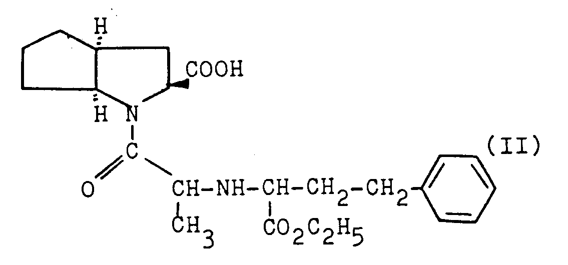
Es ist bekannt, daß Converting-Enzyme-Inhibitoren das Enzym nicht nur im Serum, sondern auch in verschiedenen Geweben, besonders auch in Herzen zu hemmen vermögen. Diese Hemmung im Herzgewebe verringert die Bildung von Angiotensin II und damit dessen nachteilige Einflüsse auf verschiedene Parameter der Herztätigkeit.

Orale Behandlung an spontan hypertonen Ratten mit einer einmaligen Dosis von 1 mg/kg hemmt das ACE im Herzen länger als 24 Stunden signifikant. Dagegen wird mit 30 mg/kg N-(1-Carbethoxy-3-phenyl-propyl)-S-alanyl-S-prolin (Enalapril) per os bereits nach mehr als 6 Stunden keine signifikante Hemmung mehr beobachtet. Chronische orale Behandlung von spontan hypertensiven Ratten über zwei Wochen mit 1 mg/kg der Verbindung der Formel II führt am Ende der Behandlung zu einer 55 %igen Hemmung des ACE im Herzen, mit 30 mg/kg Enalapril per os wird dagegen unter sonst gleichen Bedingungen keine Hemmung beobachtet. Zusätzlich führt die systemische Blutdrucksenkung, die durch Verbindungen der Formel I hervorgerufen wird, zu einer Nachlastsenkung und damit zu einer Entlastung des insuffizienten Herzens.

Postganglionäre sympathische Stimulation am isolierten Herzen führt über gesteigerte Catecholamin-Freisetzung zum Anstieg von Herzfrequenz, Kontraktionskraft und zu einem Abfall der Coronardurchströmung. Orale Vorbehandlung mit 1 mg/kg per os der Verbindung der Formel I an Kaninchen vermindert signifikant den Einfluß auf Herzfrequenz und Coronardurchströmung, läßt jedoch die Kontraktionskraft unbeeinflußt. Diese Effekte wirken zum Schutz des insuffizienten Herzens zusammen.

Angiotensin I vermindert, Bradykinin dagegen verstärkt die Coronardurchströmung am isolierten Herzen von Kaninchen, Meerschweinchen und Ratten. Orale Vorbehandlung der Tiere mit 1 mg/kg der Verbindung der Formel I peroral vermindert den Effekt von Angiotensin I und steigert die Wirkung von Bradykinin signifikant. Ein gleicher Effekt kann erst mit 30 mg/kg Enalapril erreicht werden.

Die folgenden Beispiele geben die Anwendungsformen zur Behandlung der Herzinsuffizienz nach der erfindungsgemäßen Methode an. Die Verbindungen der Formel I können analog den Beispielen in die entsprechenden Anwendungsformen gebracht werden.

### Beispiel 1

Herstellung des erfindungsgemäß verwendeten Mittels zur oralen Anwendung in der Behandlung der Herzinsuffizenz.

1000 Tabletten, die je 10 mg 1-N-(1-S-Carbethoxy-3-phenyl-propyl)-S-alanyl-1S,3S,5S-2-azabicyclo[3.3.0]octan-3-carbonsäure enthalten, werden mit den folgenden Hilfsmitteln hergestellt:

| | |
|---|---|
| N-(1-S-Carbethoxy-3-phenyl-propyl)-S-alanyl-1S,3S,5S-2-azabicyclo[3.3.0]octan-3-carbonsäure | 10 g |
| Maisstärke | 140 g |
| Gelatine | 7,5 g |
| Mikrokristalline Cellulose | 2,5 g |
| Magnesiumstearat | 2,5 g |

N-(1-S-Carbethoxy-3-phenyl-propyl)-S-alanyl-1S,3S-5S,-2-azabicyclo[3.3.0]octan-3-carbonsäure und Maisstärke werden mit einer wäßrigen Gelatine-Lösung gemischt. Die Mischung wird getrocknet und zu einem Granulat vermahlen. Mikrokristalline Cellulose und Magnesiumstearat werden mit dem Granulat vermischt. Das entstandene Granulat wird zu 1000 Tabletten gepreßt, wobei jede Tablette 10 mg des ACE-Hemmers enthält.

Diese Tabletten können zur Behandlung der Herzinsuffizenz verwendet werden.

### Beispiel 2

Gelatine-Kapseln, die je 10 mg N-(1-S-Carbethoxy-3-phenyl-propyl)-S-alanyl-1S,3S,5S-2-azabicyclo[3.3.0]octan-3-carbonsäure enthalten, werden mit der folgenden Mischung gefüllt:

| | |
|---|---|
| N-(1-S-Carbethoxy-3-phenyl-propyl)-S-alanyl-1S,3S,5S-2-azabicyclo[3.3.0]octan-3-carbonsäure | 10 mg |
| Magnesiumstearat | 1 mg |
| Lactose | 214 mg |

Diese Kapseln können zur Behandlung der Herzinsuffizienz verwendet werden.

### Beispiel 3

Die Herstellung einer Injektionslösung zur Behandlung der Herzinfuffizenz wird im folgenden beschrieben:

| | |
|---|---|
| N-(1-S-Carboxy-3-phenyl-propyl)-S-alanyl-1S,3S,5S-2-azabicyclo[3.3.0]octan-3-carbonsäure | 250 mg |
| Methylparaben | 5 g |
| Propylparaben | 1 g |
| Natriumchlorid | 25 g |
| Wasser für Injektion | 5 l |

N-(1-S-Carboxy-3-phenyl-propyl)-S-alanyl-1S,3S,5S-2-azabicyclo[3.3.0]octan-3-carbonsäure, die Konservierungsstoffe und Natriumchlorid werden in 3 l Wasser für Injektion gelöst und auf 5 l mit Wasser für Injektion aufgefüllt. Die Lösung wird steril gefiltert und aseptisch in vorsterilisierte Flaschen gefüllt, die mit sterilisierten Gummikappen verschlossen werden. Jede Flasche enthält 5 ml Lösung.

### Beispiel 4

Tabletten, die zur Behandlung der Herzinsuffizenz verwendet werden können, werden wie in Beispiel 1 beschrieben hergestellt, mit der Ausnahme, daß anstelle von N-(1-S-Carbethoxy-3-phenyl-propyl)-S-alanyl-1S,3S,5S-2-azabicyclo[3.3.0]octan-3S-carbonsäure
N-(1-S-Carboxy-3-phenyl-propyl)-S-alanyl-1S,3S,5S-2-azabicyclo[3.3.0]octan-3-carbonsäure
N-(1-S-Carboxy-3-phenyl-propyl)-S-lysyl-1S,3S,5S-2-azabicyclo[3.3.0]octan-3-carbonsäure
angewendet werden.

### Beispiel 5

Eine Injektionslösung wird analog der in Beispiel 3 beschriebenen Vorschrift hergestellt, mit der Ausnahme, daß anstelle von N-(1-S-Carbethoxy-3-phenyl-propyl)-S-alanyl-1S,3S,5S-2-azabicyclo[3.3.0]octan-3-carbonsäure
N-(1-S-Carboxy-3-phenyl-propyl)-S-alanyl-1S,3S,5S-2-azabicyclo[3.3.0]octan-3-carbonsäure oder
N-(1-Carboxy-3-phenyl-propyl)-S-lysyl-1S,3S,5S-2-azabicyclo[3.3.0]octan-3-carbonsäure
angewendet werden.

## Patentansprüche

1. Verwendung von Angiotensin-Converting-Enzyme-Inhibitoren der Formel I 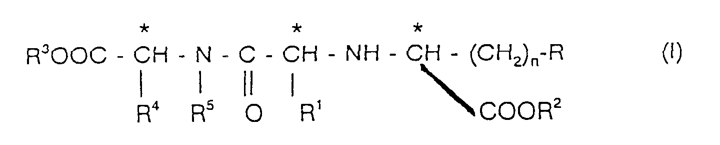 in welcher
n = 1 oder 2 ist,
R = Phenyl,
R² und R³ gleich oder verschieden Wassertoff oder Ethyl,
R¹ Wasserstoff oder C₁-C₆-Alkyl, das gegebenenfalls durch Amino substituiert sein kann, und
R⁴ und R⁵ zusammen mit den sie tragenden Atomen für ein Octahydrocyclopenta[b]pyrrol System stehen,
oder deren physiologisch verträglichen Salzen
bei der Herstellung eines Arnzeimittels zur Behandlung der Herzinsuffizienz bei Säugern.

2. Verwendung gemäß Anspruch 1, dadurch gekennzeichnet, daß (S,S,S,S,S)-N-(1-Carbethoxy 3-phenyl-propyl)-alanyl-2-azabicyclo-[3.3.0]octan-3-carbonsäure oder dessen physiologisch verträgliches Salz angewendet wird.

3. Verwendung gemäß Anspruch 1, dadurch gekennzeichnet, daß die Angiotensin-Converting-Enzyme-Inhibitoren oral oder parenteral angewendet werden.

4. Verwendung gemäß Anspruch 1, dadurch gekennzeichnet daß die Angiotensin-Converting-Enzyme-Inhibitoren für die entsprechenden Anwendungsformen mit pharmazeutisch geeigneten Trägerstoffen bzw. Hilfsstoffen kombiniert werden.

## Claims

1. Use of angiotensin-converting enzyme inhibitors of the formula I 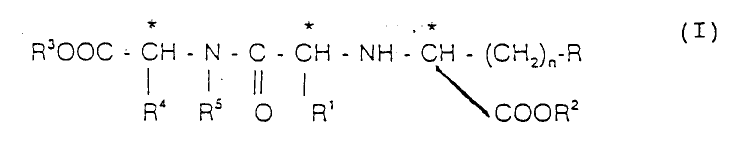 in which
n is = 1 or 2,
R is = phenyl,
R² and R³ are identical or different and are hydrogen or ethyl,
R¹ is hydrogen or C₁-C₆-alkyl, which can, where appropriate, be substituted by amino, and
R⁴ and R⁵, together with the atoms carrying them, are an octahydrocyclopenta[b)pyrrole system,
or their physiologically tolerated salts
in the preparation of a pharmaceutical for treating cardiac insufficiency in mammals.

2. The use as claimed in Claim 1, wherein (S,S,S,S,S)-N-(1-carbethoxy-3-phenylpropyl)alanyl-2-azabicyclo[3.3.0]octane-3-carboxylic acid or its physiologically tolerated salt is employed.

3. The use as claimed in Claim 1, wherein the angiotensin-converting enzyme inhibitors are employed orally or parenterally.

4. The use as claimed in Claim 1, wherein the angiotensin-converting enzyme inhibitors are combined with pharmaceutically appropriate vehicles or auxiliary substances for the corresponding use forms.

## Revendications

1. Utilisation d'inhibiteurs de l'enzyme de conversion de l'angiotensine de formule I 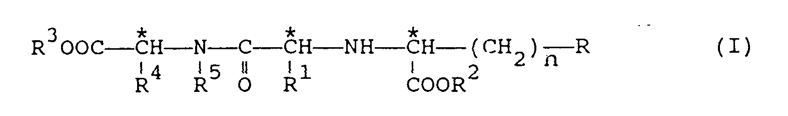 dans laquelle
n est 1 ou 2,
R représente le groupe phényle,
R² et R³ sont identiques ou différents et représentent un atome d'hydrogène ou le groupe éthyle,
R¹ représente un atome d'hydrogène ou un groupe alkyle en C₁-C₆ qui peut éventuellement être substitué par le groupe amino,
R⁴ et R⁵ forment ensemble, avec les atomes qui les portent, un système octahydrocyclopenta[b]pyrrole,
ou de leurs sels physiologiquement acceptables, dans la fabrication d'un médicament pour le traitement de l'insuffisance cardiaque chez des mammifères.

2. Utilisation selon la revendication 1, caractérisée en ce que l'on utilise l'acide (S,S,S,S,S)-N-(1-carbéthoxy-3-phénylpropyl)-alanyl-2-azabicyclo[3.3.0]octane-3-carboxylique ou un de ses sels physiologiquement acceptables.

3. Utilisation selon la revendication 1, caractérisée en ce que les inhibiteurs de l'enzyme de conversion de l'angiotensine sont utilisés par voie orale ou parentérale.

4. Utilisation selon la revendication 1, caractérisée en ce que, pour les formes d'administration correspondantes, on associe les inhibiteurs de l'enzyme de conversion de l'angiotensine à des véhicules ou adjuvants pharmaceutiques appropriés.
